# EUROPEAN PATENT SPECIFICATION

(11) **EP 3 581 114 B1**
(45) Date of publication and mention of the grant of the patent: **16.03.2022**
(21) Application number: 17895999.5
(22) Date of filing: 06.12.2017
(51) Int. Cl.: A61B 90/00, A61B 8/08, A61B 17/34

(54) **PHOTO-ACOUSTIC IMAGE GENERATION DEVICE**
FOTOAKUSTISCHE BILDERZEUGUNGSVORRICHTUNG
DISPOSITIF DE GÉNÉRATION D'IMAGE PHOTO-ACOUSTIQUE

(30) Priority: 10.02.2017 JP 2017022857
(43) Date of publication of application: 18.12.2019
(73) Proprietor: FUJIFILM Corporation, Tokyo 106-8620 (JP)
(72) Inventor: YAMAMOTO, Katsuya, Ashigarakami-gun Kanagawa 258-8538 (JP)
(74) Representative: Klunker IP Patentanwälte PartG mbB
(86) International application number: PCT/JP2017/043815
(87) International publication number: WO 2018/146929

(56) References cited:
- WO-A1-2016/047143
- JP-A- H1 133 028
- JP-A- 2012 196 308
- JP-A- 2015 231 583
- JP-A- 2016 064 011
- US-A1- 2010 298 704

## Description

### BACKGROUND OF THE INVENTION

### 1. Field of the Invention

The present invention relates to a photoacoustic image generation apparatus comprising an insert of which at least a portion is insertable into a subject and which includes a photoacoustic wave generation portion that absorbs light and generates photoacoustic waves.

### 2. Description of the Related Art

An ultrasonography method has been known as a kind of image inspection method that can non-invasively inspect the internal state of a living body. In ultrasonography, an ultrasound probe that can transmit and receive ultrasonic waves is used. In a case in which the ultrasound probe transmits ultrasonic waves to a subject (living body), the ultrasonic waves travel in the living body and are reflected from the interface between tissues. The ultrasound probe receives the reflected ultrasonic waves and a distance is calculated on the basis of the time until the reflected ultrasonic waves return to the ultrasound probe. In this way, it is possible to capture an image indicating the internal aspect of the living body.

For example, JP2013-188428A proposes an ablation therapy using a puncture needle which is performed using an ultrasound diagnostic apparatus for the above-mentioned ultrasonography. JP2013-188428A discloses a technique which determines the position of the tip of the puncture needle on the basis of positional information acquired by a magnetic sensor and displays an index indicating the position of the tip of the puncture needle so as to be superimposed on an ultrasound image.

WO2016047143A1 discloses a photoacoustic image generation device in which q puncture needle includes an outer needle and an inner needle, and the inner needle includes a light emission unit and a photoacoustic wave generation unit. An insertion/withdrawal detection means detects whether the inner needle of the puncture needle has been withdrawn from the outer needle. When it is detected that the inner needle has been withdrawn, a processing switching means changes the image display to a different image display from the image display before it was detected that the inner needle had been withdrawn.

US2010298704A1 discloses freehand ultrasound imaging systems having an ultrasound transducer equipped with a position marker and a needle equipped with a position marker allowing the position and orientation of the transducer and needle to be determined. A display depicts an acquired ultrasound image and a graphical element representative of a projection of the longitudinal axis of the needle onto a plane of the ultrasound image.

### SUMMARY OF THE INVENTION

Here, in JP2013-188428A, the position of the tip of the puncture needle is detected by the magnetic sensor or an encoder as described above. In addition, in JP2015-231583A, a photoacoustic wave generation portion provided in the vicinity of the tip of the puncture needle is irradiated with light guided by a light guide member that is provided in the puncture needle and generates photoacoustic waves. A probe detects the photoacoustic waves and generates an image such that the position of the puncture needle in the photoacoustic image is checked.

Naturally, the speed of sound in the tissue is partially different. An ultrasound image on which an index indicating the position of the tip of the puncture needle is superimposed is generated by performing an arithmetic process assuming that the speed of sound in the tissue is constant. Therefore, the actual position in the tissue deviates from the position in the ultrasound image. That is, in a case in which the position of the tip of the puncture needle detected by, for example, the magnetic sensor is displayed so as to be superimposed on the ultrasound image as described above, the position of the tip is not correct.

Further, in JP2013-188428A, it is necessary to perform synchronization in order to align the time phase of a sensing mechanism provided in the puncture needle with the time phase of the frame of the ultrasound image and it is difficult to display the detected position of the tip of the puncture needle in real time.

The invention has been made in view of the above-mentioned problems and an object of the invention is to provide a photoacoustic image generation apparatus that can detect a position of a tip of an insert, such as a puncture needle, which can be inserted into a subject on an ultrasound image with high accuracy and can detect a distance between the position of the tip of the insert and a measurement point on the ultrasound image with high accuracy.

According to an aspect of the invention, there is provided a photoacoustic image generation apparatus according to claim 1 of the appended claims.

The photoacoustic image generation apparatus according to the aspect of the invention may further comprise a feature point detection unit that detects a feature point on the basis of the reflected acoustic image. The measurement unit may measure a first distance between the position of the tip of the insert and the designated measurement point and a second distance between the position of the tip of the insert and the feature point.

The photoacoustic image generation apparatus according to the aspect of the invention may further comprise a feature point detection unit that detects a feature point on the basis of the reflected acoustic image. The measurement unit may measure the distance, using the feature point as the measurement point.

The photoacoustic image generation apparatus according to the aspect of the invention may further comprise a movement detection unit that detects movement of the position of the tip of the insert, using the reflected acoustic image and the photoacoustic image.

In the photoacoustic image generation apparatus according to the aspect of the invention, the measurement unit may remeasure the distance in a case in which the movement detection unit detects the movement of the position of the tip of the insert.

In the photoacoustic image generation apparatus according to the aspect of the invention, the probe may alternately detect the photoacoustic waves and the reflected acoustic waves.

According to an aspect of the invention, there is provided a photoacoustic image generation apparatus according to claim 7 of the appended claims.

According to the photoacoustic image generation apparatus of the aspect of the invention, the probe detects the photoacoustic waves generated from the photoacoustic wave generation portion provided in the tip portion of the insert and the reflected acoustic waves reflected by the transmission of the acoustic waves to the subject and the position of the tip of the insert is detected on the basis of the photoacoustic image generated on the basis of the detected photoacoustic waves. Therefore, it is possible to detect the position of the tip of the insert on the ultrasound image with high accuracy. Then, the distance between the position of the tip of the insert detected on the photoacoustic image and the measurement point on the reflected acoustic image is measured. Therefore, it is possible to measure the distance between the position of the tip of the insert and the measurement point with high accuracy.

### BRIEF DESCRIPTION OF THE DRAWINGS

Fig. 1 is a block diagram schematically illustrating the configuration of a first embodiment of a photoacoustic image generation apparatus according to the invention.
Fig. 2 is a cross-sectional view illustrating the configuration of a tip portion of a puncture needle.
Fig. 3 is a flowchart illustrating the operation of the first embodiment of the photoacoustic image generation apparatus according to the invention.
Fig. 4 is a diagram illustrating an example of the position of a tip of the puncture needle and a designated measurement point on an ultrasound image.
Fig. 5 is a block diagram schematically illustrating the configuration of a second embodiment of the photoacoustic image generation apparatus according to the invention.
Fig. 6 is a diagram illustrating an example of the position of the tip of the puncture needle and a feature point on the ultrasound image.
Fig. 7 is a flowchart illustrating the operation of the second embodiment of the photoacoustic image generation apparatus according to the invention.
Fig. 8 is a block diagram schematically illustrating the configuration of a third embodiment of the photoacoustic image generation apparatus according to the invention.
Fig. 9 is a flowchart illustrating the operation of the third embodiment of the photoacoustic image generation apparatus according to the invention.
Fig. 10 is a diagram illustrating an example of the position of the tip of the puncture needle, a designated measurement point, and a feature point on the ultrasound image.
Fig. 11 is a flowchart illustrating the operation of a modification example of the first embodiment of the photoacoustic image generation apparatus according to the invention.
Fig. 12 is a block diagram schematically illustrating the configuration of a modification example in which a movement detection unit is provided in the first embodiment of the photoacoustic image generation apparatus according to the invention.
Fig. 13 is a flowchart illustrating the operation of the modification example illustrated in Fig. 12.

### DESCRIPTION OF THE PREFERRED EMBODIMENTS

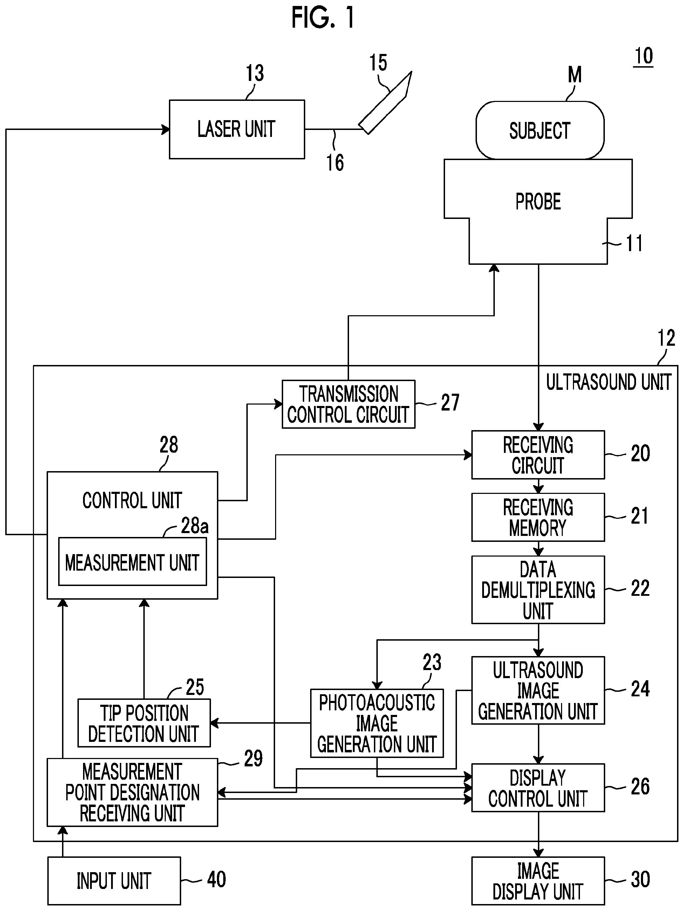

Hereinafter, a first embodiment of a photoacoustic image generation apparatus according to the invention will be described in detail with reference to the drawings. Fig. 1 is a block diagram schematically illustrating the configuration of a photoacoustic image generation apparatus 10 according to this embodiment.

As illustrated in Fig. 1, the photoacoustic image generation apparatus 10 according to this embodiment comprises a probe 11, an ultrasound unit 12, a laser unit 13, and a puncture needle 15. The puncture needle 15 and the laser unit 13 are connected by an optical cable 16 having an optical fiber. The puncture needle 15 can be attached to and detached from the optical cable 16 and is disposable. In addition, in this embodiment, ultrasonic waves are used as acoustic waves. However, the invention is not limited to the ultrasonic waves. Acoustic waves with an audible frequency may be used as long as an appropriate frequency can be selected according to, for example, an inspection target or measurement conditions.

The laser unit 13 comprises a solid-state laser light source using, for example, yttrium aluminum garnet (YAG) and alexandrite. Laser light emitted from the solid-state laser light source of the laser unit 13 is guided by the optical cable 16 and is incident on the puncture needle 15. The laser unit 13 according to this embodiment emits pulsed laser light in a near-infrared wavelength range. The near-infrared wavelength range means a wavelength range from about 700 nm to 2500 nm. In this embodiment, the solid-state laser light source is used. However, other laser light sources, such as a gas laser light source, may be used or light sources other than the laser light source may be used.

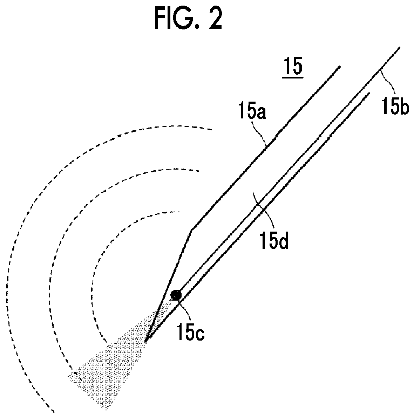

The puncture needle 15 is an embodiment of an insert according to the invention and is a needle that is inserted into a subject M. Fig. 2 is a cross-sectional view including a center axis that extends in a length direction of the puncture needle 15. The puncture needle 15 includes a puncture needle main body 15a that has an opening at an acute tip and is formed in a hollow shape, an optical fiber 15b (corresponding to a light guide member according to the invention) that guides laser light emitted from the laser unit 13 to the vicinity of the opening of the puncture needle 15, and a photoacoustic wave generation portion 15c that absorbs laser light emitted from the optical fiber 15b and generates photoacoustic waves.

The optical fiber 15b and the photoacoustic wave generation portion 15c are provided in a hollow portion 15d of the puncture needle main body 15a. For example, the optical fiber 15b is connected to the optical fiber in the optical cable 16 (see Fig. 1) through an optical connector that is provided at the base end of the puncture needle 15. For example, a laser light of 0.2 mJ is emitted from a light emission end of the optical fiber 15b.

The photoacoustic wave generation portion 15c is provided at the light emission end of the optical fiber 15b and is provided in the vicinity of the tip of the puncture needle 15 and in the inner wall of the puncture needle main body 15a. The photoacoustic wave generation portion 15c absorbs the laser light emitted from the optical fiber 15b and generates photoacoustic waves. The photoacoustic wave generation portion 15c is made of, for example, an epoxy resin, a polyurethane resin, a fluorine resin, and silicone rubber with which a black pigment is mixed. In Fig. 2, the photoacoustic wave generation portion 15c is illustrated to be larger than the optical fiber 15b. However, the invention is not limited thereto. The photoacoustic wave generation portion 15c may have a size that is equal to the diameter of the optical fiber 15b.

The photoacoustic wave generation portion 15c is not limited to the above and a metal film or an oxide film having light absorptivity with respect to the wavelength of laser light may be used as the photoacoustic wave generation portion. An oxide film made of, for example, iron oxide, chromium oxide, or manganese oxide having high light absorptivity with respect to the wavelength of laser light can be used as the photoacoustic wave generation portion 15c. Alternatively, a metal film made of, for example, titanium (Ti) or platinum (Pt) that has a lower light absorptivity than an oxide and has a higher biocompatibility than an oxide may be used as the photoacoustic wave generation portion 15c. In addition, the position where the photoacoustic wave generation portion 15c is provided is not limited to the inner wall of the puncture needle main body 15a. For example, a metal film or an oxide film which is the photoacoustic wave generation portion 15c may be formed on the light emission end of the optical fiber 15b with a thickness of about 100 nm by vapor deposition such that the oxide film covers the light emission end. In this case, at least a portion of the laser light emitted from the light emission end of the optical fiber 15b is absorbed by the metal film or the oxide film covering the light emission end and photoacoustic waves are generated from the metal film or the oxide film.

The vicinity of the tip of the puncture needle 15 means a position where the photoacoustic wave generation portion 15c can generate photoacoustic waves capable of imaging the position of the tip of the puncture needle 15 with accuracy required for a needling operation in a case in which the tip of the optical fiber 15b and the photoacoustic wave generation portion 15c are disposed at the position. For example, the vicinity of the tip of the puncture needle 15 is the range of 0 mm to 3 mm from the tip to the base end of the puncture needle 15. In the subsequent embodiments, the meaning of the vicinity of the tip is the same as described above.

Returning to Fig. 1, the probe 11 is an ultrasound probe and includes, for example, a plurality of ultrasound transducers which are one-dimensionally or two-dimensionally arranged. The ultrasound transducer is, for example, a piezoelectric element made of a polymer film, such as piezoelectric ceramics or polyvinylidene fluoride (PVDF).

The probe 11 detects the photoacoustic waves generated from the photoacoustic wave generation portion 15c after the puncture needle 15 is inserted into a subject M. In addition, the probe 11 performs the transmission of ultrasonic waves (acoustic waves) to the subject M and the detection of reflected ultrasonic waves (reflected acoustic waves) with respect to the transmitted ultrasonic waves, in addition to the detection of the photoacoustic waves.

The probe 11 according to this embodiment alternately detects the photoacoustic waves and the reflected ultrasonic waves. Therefore, frames of photoacoustic images and frames of ultrasound images are alternately generated.

In addition, the transmission and reception of the ultrasonic waves may be performed at different positions. For example, ultrasonic waves may be transmitted from a position different from the position of the probe 11 and the probe 11 may receive the reflected ultrasonic waves with respect to the transmitted ultrasonic waves. For example, a linear ultrasound probe, a convex ultrasound probe, or a sector ultrasound probe may be used as the probe 11.

The ultrasound unit 12 includes a receiving circuit 20, a receiving memory 21, a data demultiplexing unit 22, a photoacoustic image generation unit 23, an ultrasound image generation unit 24, a tip position detection unit 25, a display control unit 26, a transmission control circuit 27, a control unit 28, and a measurement point designation receiving unit 29. The ultrasound unit 12 typically includes, for example, a processor, a memory, and a bus. A program related to, for example, a photoacoustic image generation process, an ultrasound image generation process, a process of detecting the position of the tip of the puncture needle 15 in a photoacoustic image, and a measurement process which will be described below is incorporated into a memory in the ultrasound unit 12. The program is executed by the control unit 28 which is formed by a processor to implement the functions of the data demultiplexing unit 22, the photoacoustic image generation unit 23, the ultrasound image generation unit 24, the tip position detection unit 25, the display control unit 26, the measurement unit 28a, and the measurement point designation receiving unit 29. That is, each of these units is formed by the processor and the memory into which the program has been incorporated.

The hardware configuration of the ultrasound unit 12 is not particularly limited and can be implemented by an appropriate combination of, for example, a plurality of integrated circuits (ICs), a processor, an application specific integrated circuit (ASIC), a field-programmable gate array (FPGA), and a memory.

The receiving circuit 20 receives a detection signal output from the probe 11 and stores the received detection signal in the receiving memory 21. The receiving circuit 20 typically includes a low-noise amplifier, a variable-gain amplifier, a low-pass filter, and an analog-to-digital converter (AD converter). The detection signal of the probe 11 is amplified by the low-noise amplifier. Then, gain adjustment corresponding to the depth of the tip of the puncture needle 15 is performed by the variable-gain amplifier and a high-frequency component of the detection signal is cut by the low-pass filter. Then, the detection signal is converted into a digital signal by the AD converter and the digital signal is stored in the receiving memory 21. The receiving circuit 20 is formed by, for example, one integral circuit (IC).

The probe 11 outputs a detection signal of the photoacoustic waves and a detection signal of the reflected ultrasonic waves. The AD-converted detection signals (sampling data) of the photoacoustic waves and the reflected ultrasonic waves are stored in the receiving memory 21.

In a case in which a photoacoustic image is generated, the data demultiplexing unit 22 reads the detection signal of the photoacoustic waves from the receiving memory 21 and transmits the detection signal to the photoacoustic image generation unit 23. In addition, in a case in which an ultrasound image is generated, the data demultiplexing unit 22 reads the detection signal of the reflected ultrasonic waves from the receiving memory 21 and transmits the detection signal to the ultrasound image generation unit 24.

The photoacoustic image generation unit 23 generates a photoacoustic image on the basis of the detection signal of the photoacoustic waves detected by the probe 11. The photoacoustic image generation process includes image reconstruction, such as phasing addition, detection, and logarithmic conversion.

The ultrasound image generation unit 24 (corresponding to a reflected acoustic image generation unit according to the invention) generates an ultrasound image (reflected acoustic image) on the basis of the detection signal of the reflected ultrasonic waves detected by the probe 11. The ultrasound image generation process includes image reconstruction, such as phasing addition, detection, and logarithmic conversion. In addition, a sound speed used in the ultrasound image generation process is equal to a sound speed used in the photoacoustic image generation process.

The tip position detection unit 25 detects the position of the tip of the puncture needle 15 on the basis of the photoacoustic image generated by the photoacoustic image generation unit 23. As a method for detecting the position of the tip of the puncture needle 15, any method may be used as long as it can detect the position of a maximum brightness point in the photoacoustic image as the position of the tip of the puncture needle 15.

In a case in which the position of the tip of the puncture needle 15 is detected on the basis of the photoacoustic image as described above, in practice, an artifact of light or an artifact of sound is generated and a photoacoustic image in which photoacoustic waves are detected from a plurality of positions is likely to be generated. As a result, the original position of the tip of the puncture needle 15 is unlikely to be specified.

For this reason, the photoacoustic image generated by the photoacoustic image generation unit 23 is not used as it is, but, for example, a smoothing process may be performed for the photoacoustic image to prevent erroneous detection caused by the artifact. Specifically, the smoothing process is performed for the photoacoustic image subjected to detection and logarithmic conversion. For example, a filtering process using a Gaussian filter can be used as the smoothing process. It is preferable that the size of the Gaussian filter is less than that of the tip portion of the puncture needle 15.

Then, a binarization process is performed for the photoacoustic image subjected to the smoothing process to generate a binary image. Then, a region in which white pixels are continuously distributed is detected from the binary image to detect the position of the tip of the puncture needle 15. In this way, it is possible to detect the position of the tip of the puncture needle 15 with higher accuracy.

In this embodiment, information of the position of the tip of the puncture needle 15 detected by the tip position detection unit 25 is output to the display control unit 26 and a measurement unit 28a.

The display control unit 26 displays the photoacoustic image and the ultrasound image on an image display unit 30 including, for example, a display device. In addition, the display control unit 26 displays an index indicating the position of the tip of the puncture needle 15 detected on the basis of the photoacoustic image and an index indicating a measurement point on the ultrasound image which has been designated by the user on the ultrasound image. Further, the ultrasound image and the photoacoustic image may be displayed so as to be superimposed on each other.

The control unit 28 controls each component in the ultrasound unit 12. For example, in a case in which a photoacoustic image is acquired, the control unit 28 transmits a trigger signal to the laser unit 13 such that the laser unit 13 emits pulsed laser light. In addition, the control unit 28 transmits a sampling trigger signal to the receiving circuit 20 to control, for example, the sampling start time of the photoacoustic waves with the emission of the laser light. The detection signal of the photoacoustic waves which has been received by the receiving circuit 20 and then converted into a digital signal is stored in the receiving memory 21.

In a case in which an ultrasound image is acquired, the control unit 28 transmits an ultrasound transmission trigger signal for commanding the transmission of ultrasonic waves to the transmission control circuit 27. In a case in which the ultrasound transmission trigger signal is received, the transmission control circuit 27 directs the probe 11 to transmit ultrasonic waves. The control unit 28 transmits the sampling trigger signal to the receiving circuit 20 according to the transmission time of ultrasonic waves such that the receiving circuit 20 starts the sampling of the reflected ultrasonic waves. The detection signal of the ultrasonic waves which has been received by the receiving circuit 20 and then converted into a digital signal is stored in the receiving memory 21.

The control unit 28 comprises the measurement unit 28a. The measurement unit 28a performs a measurement process of measuring the distance between the position of the tip of the puncture needle 15 detected in the photoacoustic image and the measurement point designated by the user on the ultrasound image.

The measurement point designation receiving unit 29 acquires the positional information of the measurement point designated by the user on the ultrasound image. Specifically, the user designates the measurement point on the ultrasound image displayed on the image display unit 30 with an input unit 40. The positional information of the measurement point input from the input unit 40 is acquired by the measurement point designation receiving unit 29 and is output to the measurement unit 28a. In addition, the input unit 40 comprises, for example, a mouse or a keyboard.

The measurement unit 28a receives the positional information of the measurement point acquired by the measurement point designation receiving unit 29 and the positional information of the tip of the puncture needle 15 output from tip position detection unit 25 and measures the distance between the measurement point and the position of the tip of the puncture needle 15 on the basis of the positional information.

In this embodiment, as described above, since photoacoustic waves and reflected ultrasonic waves are detected by the common probe 11, the coordinate axes of the photoacoustic image and the ultrasound image can be common to each other. Since the tip position detection unit 25 detects the position of the tip of the puncture needle 15 using the photoacoustic image, it is possible to detect the position of the tip of the puncture needle 15 on the ultrasound image with high accuracy. Therefore, it is possible to measure the distance between the position of the tip of the puncture needle 15 and a measurement point on the ultrasound image with high accuracy.

In this embodiment, as described above, the probe 11 alternately detects the photoacoustic waves and the reflected ultrasonic waves and alternately generates a frame of a photoacoustic image and a frame of an ultrasound image. Therefore, in a case in which the position of the tip of the puncture needle 15 is detected using a photoacoustic image of a frame immediately before a frame of an ultrasound image, it is possible to detect the position of the tip of the puncture needle 15 on the ultrasound image in real time with higher accuracy.

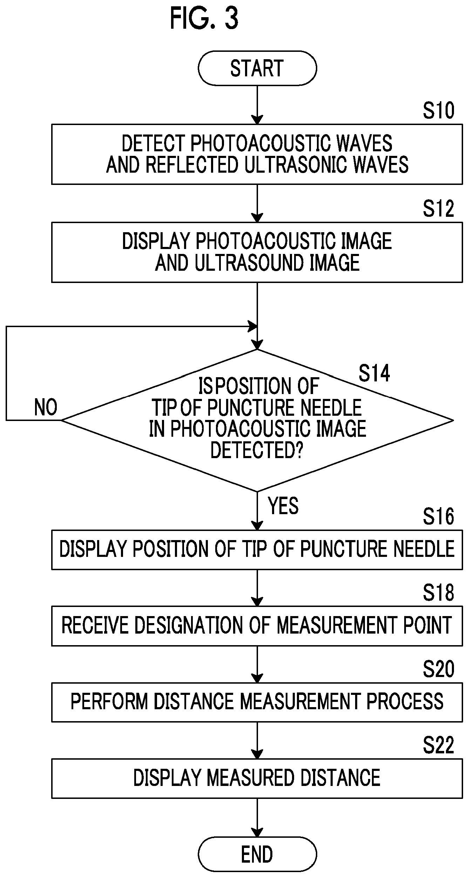

Next, the operation of the photoacoustic image generation apparatus 10 according to this embodiment will be described with reference to a flowchart illustrated in Fig. 3.

First, the common probe 11 detects the photoacoustic waves generated from the tip of the puncture needle 15 and the reflected ultrasonic waves reflected from the subject M (S10). Then, a photoacoustic image based on a detection signal of the photoacoustic waves and an ultrasound image based on a detection signal of the reflected ultrasonic waves are generated and the display control unit 26 displays the images on the image display unit 30 (S12).

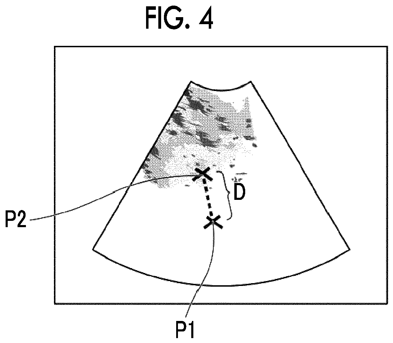

Then, the tip position detection unit 25 performs a process of detecting the position of the tip of the puncture needle 15 in the photoacoustic image. In a case in which the position of the tip of the puncture needle 15 is detected (S14, YES), an index indicating the position of the tip is displayed (S16). Specifically, a cross mark indicating a tip position P1 of the puncture needle 15 illustrated in Fig. 4 is displayed on the ultrasound image.

Then, in a case in which the position of the tip of the puncture needle 15 is detected as described above, the measurement point designation receiving unit 29 starts to receive the designation of a measurement point (S18). Specifically, in a case in which the user designates a point on the ultrasound image with the input unit 40, the measurement point designation receiving unit 29 recognizes the designated point as the measurement point and acquires the positional information of the measurement point. In a case in which the position of the tip of the puncture needle 15 is not detected on the photoacoustic image (S14, NO), the measurement point designation receiving unit 29 does not receive the designation of the measurement point by the user. Specifically, in a case in which the position of the tip of the puncture needle 15 is not detected even though the user designates a point on the ultrasound image with the input unit 40, the measurement point designation receiving unit 29 does not recognize the point as the measurement point. In other words, the measurement point designation receiving unit 29 receives the designation of the measurement point only in a case in which the position of the tip of the puncture needle 15 is detected on the photoacoustic image. This configuration makes it possible to prevent the unnecessary designation of the measurement point.

Then, the positional information of the measurement point received by the measurement point designation receiving unit 29 is output to the display control unit 26 and the display control unit 26 displays a cross mark indicating a measurement point P2 illustrated in Fig. 4 on the ultrasound image.

Then, the measurement unit 28a acquires the information of the tip position P1 of the puncture needle 15 and the positional information of the measurement point P2 designated by the user and measures a distance D (sec Fig. 4) between the tip position P1 of the puncture needle 15 and the measurement point P2 (S20).

The distance D measured by the measurement unit 28a is output to the display control unit 26 and the display control unit 26 displays the measured distance D as text on the image display unit 30 (S22).

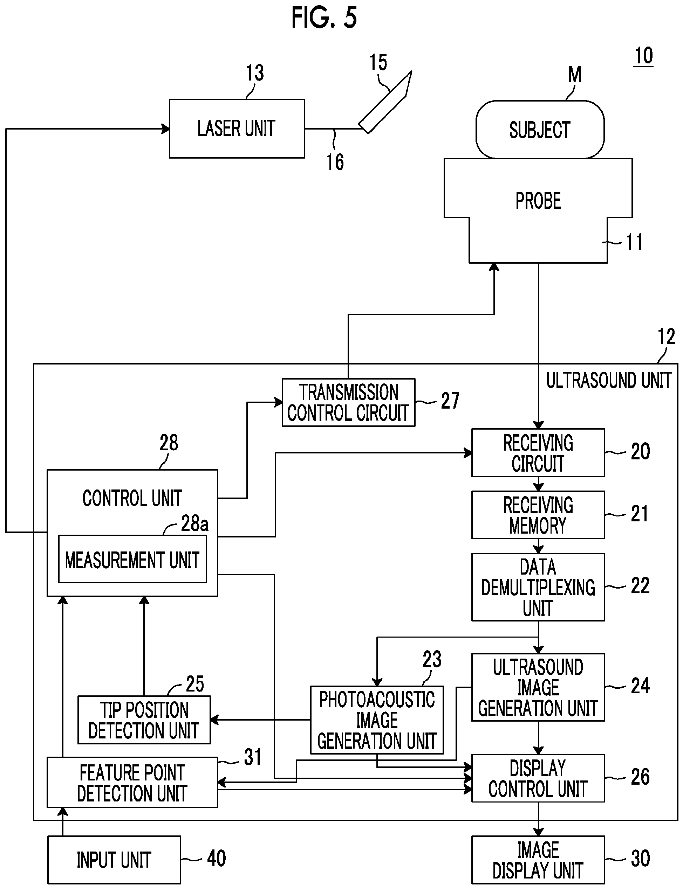

Next, a second embodiment of the photoacoustic image generation apparatus 10 according to the invention will be described. Fig. 5 is a block diagram schematically illustrating the configuration of the photoacoustic image generation apparatus 10 according to the second embodiment. The photoacoustic image generation apparatus 10 according to the first embodiment is configured such that the user designates the measurement point. However, the photoacoustic image generation apparatus 10 according to the second embodiment automatically detects the measurement point. Therefore, it is possible to reduce the time and effort required for the user to designate the measurement point and to more appropriately specify the measurement point.

As illustrated in Fig. 5, the photoacoustic image generation apparatus 10 according to the second embodiment comprises a feature point detection unit 31 instead of the measurement point designation receiving unit 29 according to the first embodiment. The other configurations are the same as those in the photoacoustic image generation apparatus 10 according to the first embodiment.

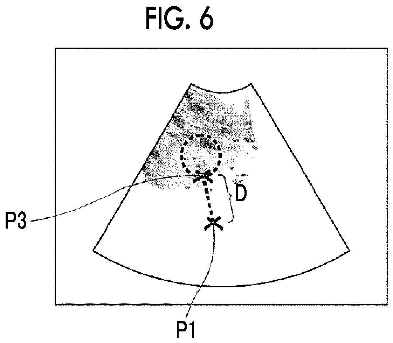

The feature point detection unit 31 according to this embodiment detects a point on a lesion included in an ultrasound image as a feature point. Examples of the lesion include a mass and a cyst. In Fig. 6, the lesion detected by the feature point detection unit 31 is represented by a dotted circle. For example, the feature point detection unit 31 detects a point that is closest to the tip position P1 of the puncture needle 15 among points on the circle indicating the lesion as a measurement point P3. The feature point is not limited to a point on the lesion and anatomic feature points, such as a predetermined blood vessel and a nerve bundle, may be detected as the feature points.

In the photoacoustic image generation apparatus 10 according to the second embodiment, the measurement unit 28a measures a distance D between the measurement point P3 detected by the feature point detection unit 31 as described above and a tip position P1 of the puncture needle 15.

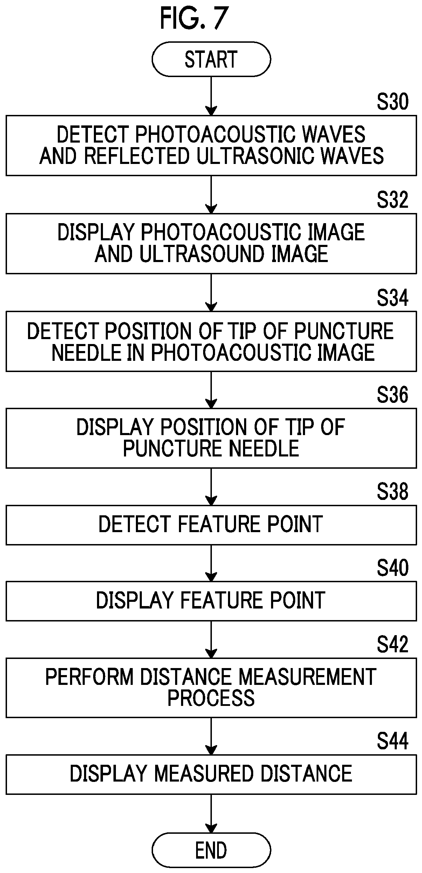

Next, the operation of the photoacoustic image generation apparatus 10 according to the second embodiment will be described with reference to a flowchart illustrated in Fig. 7.

First, similarly to the first embodiment, the probe 11 detects the photoacoustic waves generated from the tip of the puncture needle 15 and the reflected ultrasonic waves reflected from the subject M (S30). Then, a photoacoustic image based on a detection signal of the photoacoustic waves and an ultrasound image based on a detection signal of the reflected ultrasonic waves are generated and the display control unit 26 displays the images on the image display unit 30 (S32).

Then, the tip position detection unit 25 performs a process of detecting the position of the tip of the puncture needle 15 in the photoacoustic image (S34) and an index indicating the position of the tip is displayed (S36). Specifically, a cross mark indicating the tip position P1 of the puncture needle 15 illustrated in Fig. 6 is displayed on the ultrasound image.

Then, the feature point detection unit 31 detects a lesion included in the ultrasound image and detects one point on the lesion as the feature point (S38). Then, the positional information of the feature point is output to the display control unit 26 and the display control unit 26 displays a cross mark indicating a measurement point (feature point) P3 illustrated in Fig. 6 on the ultrasound image (S40).

Then, the measurement unit 28a acquires the information of the tip position P1 of the puncture needle 15 and the positional information of the measurement point P3 and measures the distance D (see Fig. 6) between the tip position P1 of the puncture needle 15 and the measurement point P3 (S42).

The distance D measured by the measurement unit 28a is output to the display control unit 26 and the display control unit 26 displays the measured distance D as text on the image display unit 30 (S44).

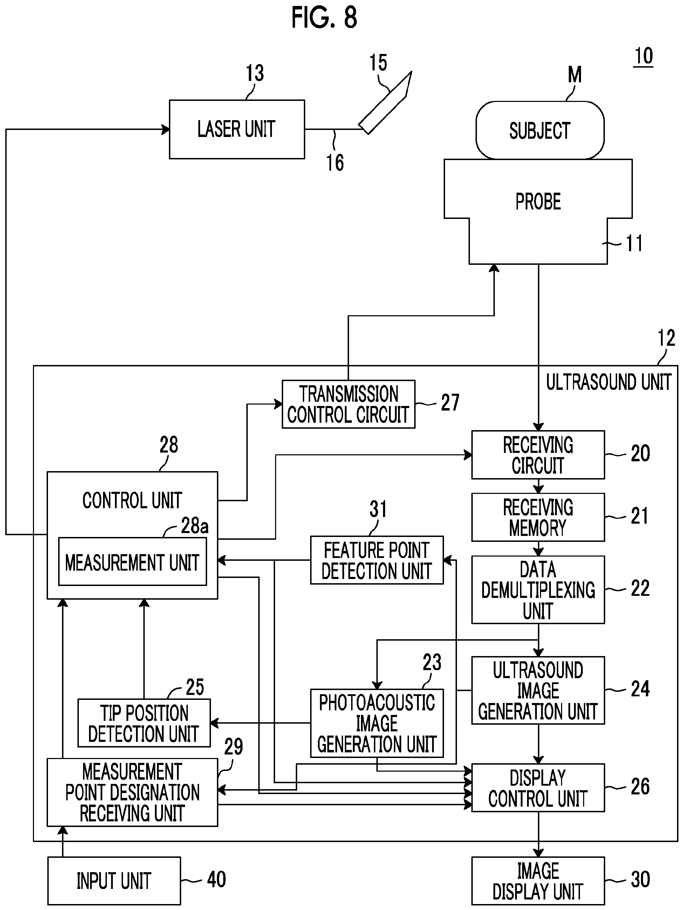

Next, a third embodiment of the photoacoustic image generation apparatus 10 according to the invention will be described. Fig. 8 is a block diagram schematically illustrating the configuration of the photoacoustic image generation apparatus 10 according to the third embodiment. The configuration of the photoacoustic image generation apparatus 10 according to the third embodiment is substantially a combination of the configuration of the photoacoustic image generation apparatus 10 according to the first embodiment and the configuration of the photoacoustic image generation apparatus 10 according to the second embodiment. That is, as illustrated in Fig. 8, the photoacoustic image generation apparatus 10 according to the third embodiment comprises both the measurement point designation receiving unit 29 and the feature point detection unit 31. The other configurations are the same as those in the photoacoustic image generation apparatuses 10 according to the first and second embodiments.

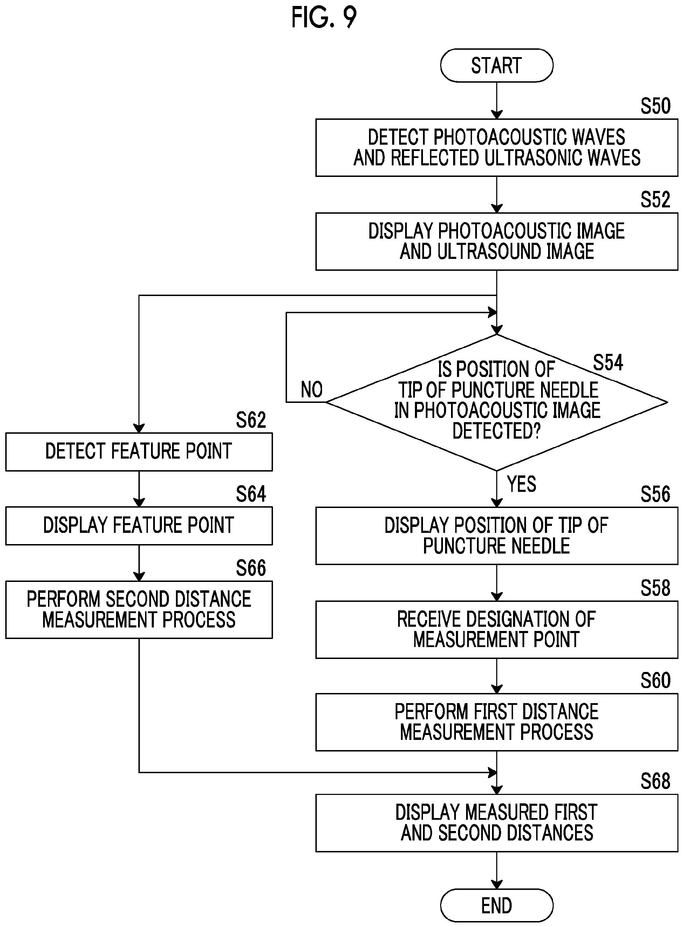

Next, the operation of the photoacoustic image generation apparatus 10 according to this embodiment will be described with reference to a flowchart illustrated in Fig. 9.

First, similarly to the first and second embodiments, the probe 11 detects the photoacoustic waves generated from the tip of the puncture needle 15 and the reflected ultrasonic waves reflected from the subject M (S50). Then, a photoacoustic image based on a detection signal of the photoacoustic waves and an ultrasound image based on a detection signal of the reflected ultrasonic waves are generated and the display control unit 26 displays the images on the image display unit 30 (S52).

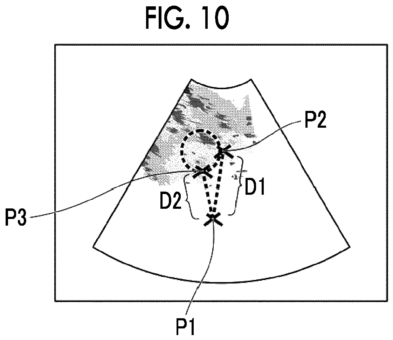

Then, the tip position detection unit 25 performs a process of detecting the position of the tip of the puncture needle 15 in the photoacoustic image. In a case in which the position of the tip of the puncture needle 15 is detected (S54, YES), an index indicating the position of the tip is displayed (S56). Specifically, a cross mark indicating a tip position P1 of the puncture needle 15 illustrated in Fig. 10 is displayed on the ultrasound image.

Then, in a case in which the position of the tip of the puncture needle 15 is detected as described above, the measurement point designation receiving unit 29 starts to receive the designation of a measurement point (S58). Specifically, in a case in which the user designates a point on the ultrasound image with the input unit 40, the measurement point designation receiving unit 29 recognizes the designated point as the measurement point and acquires the positional information of the measurement point. In a case in which the position of the tip of the puncture needle 15 is not detected on the photoacoustic image (S54, NO), the measurement point designation receiving unit 29 does not receive the designation of the measurement point by the user similarly to the first embodiment. Specifically, in a case in which the position of the tip of the puncture needle 15 is not detected even though the user designates a point on the ultrasound image with the input unit 40, the measurement point designation receiving unit 29 does not recognize the point as the measurement point. In other words, the measurement point designation receiving unit 29 receives the designation of the measurement point only in a case in which the position of the tip of the puncture needle 15 is detected on the photoacoustic image.

Then, the positional information of the measurement point received by the measurement point designation receiving unit 29 is output to the display control unit 26 and the display control unit 26 displays a cross mark indicating a measurement point P2 illustrated in Fig. 10 on the ultrasound image.

Then, the measurement unit 28a acquires the information of the tip position P1 of the puncture needle 15 and the positional information of the measurement point P2 designated by the user and measures a first distance D1 (see Fig. 10) between the tip position P1 of the puncture needle 15 and the measurement point P2 (S60).

The first distance D1 measured by the measurement unit 28a is output to the display control unit 26 and the display control unit 26 displays the measured first distance D1 as text on the image display unit 30 (S68).

After the ultrasound image is displayed in S52, the detection of a feature point by the feature point detection unit 31 is performed in parallel to the process of detecting the position of the tip of the puncture needle 15 included in the photoacoustic image (S62). Specifically, the feature point detection unit 31 detects a lesion included in the ultrasound image and one point on the lesion is detected as the feature point. Then, the positional information of the feature point is output to the display control unit 26 and the display control unit 26 displays a cross mark indicating a measurement point (feature point) P3 illustrated in Fig. 10 on the ultrasound image (S64).

Then, the measurement unit 28a acquires the information of the tip position P1 of the puncture needle 15 and the positional information of the measurement point P3 and measures a second distance D2 (see Fig. 10) between the tip position P1 of the puncture needle 15 and the measurement point P3 (S66).

The second distance D2 measured by the measurement unit 28a is output to the display control unit 26 and the display control unit 26 displays the measured second distance D2 as text on the image display unit 30 (S68). In a case in which the position of the tip of the puncture needle 15 is not detected in the photoacoustic image, only the second distance D2 is displayed as text on the image display unit 30.

Here, in the photoacoustic image generation apparatuses 10 according to the first to third embodiments which detect the position of the tip of the puncture needle 15 using the photoacoustic image and measure the distance between the position of the tip and the measurement point, in a case in which the photoacoustic image is acquired by a so-called crossing method and the tip of the puncture needle 15 is present immediately below the probe 11, the positional relationship between the position of the tip of the puncture needle 15 in the photoacoustic image and the measurement point is matched with the actual positional relationship. Therefore, the distance measured by the measurement unit 28a is a correct value. In a case in which the measurement result is continuously displayed until the tip of the puncture needle 15 passes through a portion immediately below the probe 11, the measurement result is not a correct distance and an erroneous diagnosis is likely to be made. In the crossing method, piezoelectric elements of the probe 11 are arranged along a direction orthogonal to the length direction of the puncture needle 15 and needling is performed. In a photoacoustic image acquired by a parallel method, an image of the puncture needle 15 appears linearly. In a photoacoustic image acquired by the crossing method, an image of the puncture needle 15 appears as dots.

Therefore, in the photoacoustic image generation apparatuses 10 according to the first to third embodiments, after the tip of the puncture needle 15 passes through a portion immediately below the probe 11, the measurement result may not be displayed.

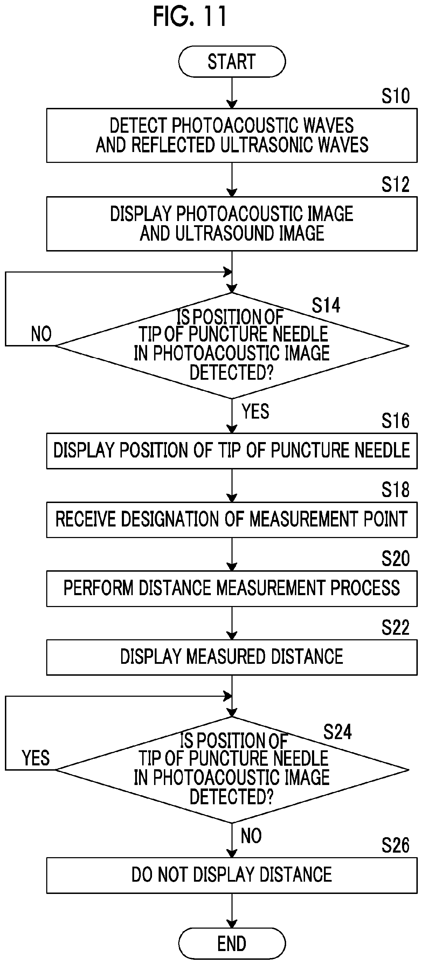

Fig. 11 is a flowchart illustrating a case in which the measurement result is not displayed in the photoacoustic image generation apparatus 10 according to the first embodiment as described above.

A process from S10 to S22 illustrated in Fig. 11 is the same as that in the first embodiment. Then, after the measured distance is displayed in S22, the tip position detection unit 25 detects the position of the tip of the puncture needle 15 on the photoacoustic image. In a case in which the position of the tip of the puncture needle 15 is detected on the photoacoustic image (S24, YES), the distance of the measurement result is continuously displayed. On the other hand, in a case in which the position of the tip of the puncture needle 15 is not detected on the photoacoustic image (S24, NO), that is, in a case in which the tip of the puncture needle 15 has passed through a portion immediately below the probe 11, the distance of the measurement result which is currently displayed is not displayed (S26). Therefore, it is possible to prevent, for example, a doctor from making an erroneous diagnosis.

In the above description, in the photoacoustic image generation apparatuses 10 according to the second and third embodiments, similarly, in a case in which the position of the tip of the puncture needle 15 is not detected on the photoacoustic image, the distance of the measurement result which is currently displayed may not be displayed.

In the photoacoustic image generation apparatuses 10 according to the first to third embodiments, the measurement of the distance between the position of the tip of the puncture needle 15 and the measurement point may not necessarily be performed for each frame of the photoacoustic image and the ultrasound image. The reason is that, in a case in which the distance measurement is performed even though the position of the tip of the puncture needle 15 is not changed, the measurement result is the same and an arithmetic process is unnecessarily performed. Therefore, the photoacoustic image generation apparatuses 10 according to the first to third embodiments may detect whether the position of the tip of the puncture needle 15 has been moved in the subject M. In a case in which the position of the tip of the puncture needle 15 has been moved, the photoacoustic image generation apparatuses 10 may remeasure the distance and update the display of the measurement result.

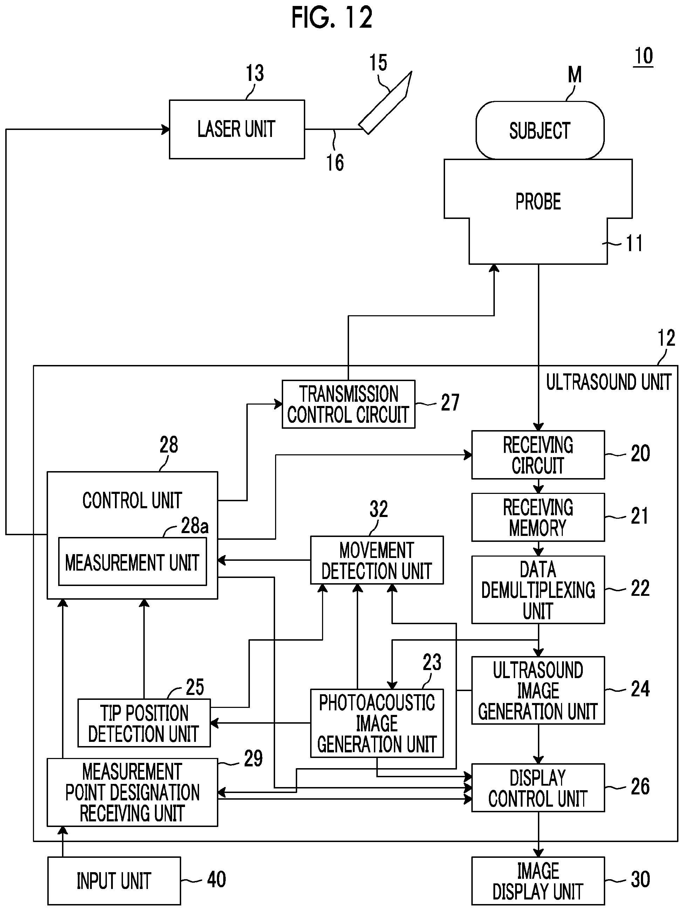

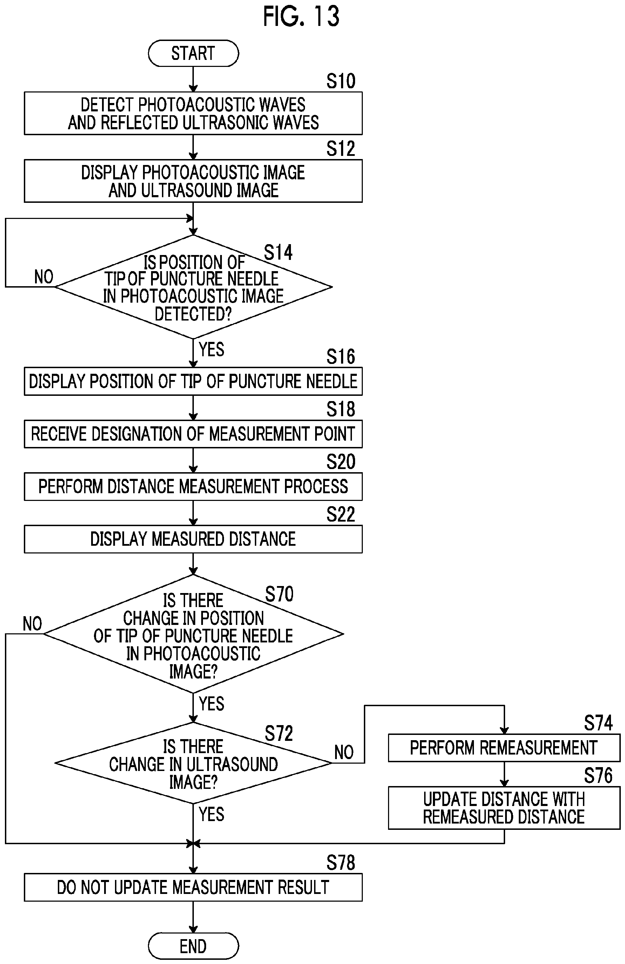

Fig. 12 is a block diagram illustrating a modification example of the photoacoustic image generation apparatus 10 according to the first embodiment in which the distance is remeasured only in a case in which the position of the tip of the puncture needle 15 has been moved in the subject M. Fig. 13 is a flowchart illustrating an operation of the modification example. As illustrated in Fig. 12, in the modification example of the first embodiment, the photoacoustic image generation apparatus 10 further comprises a movement detection unit 32 that detects the movement of the position of the tip of the puncture needle 15. The other configurations are the same as those in the first embodiment.

A process from S10 to S22 illustrated in Fig. 13 is the same as that in the first embodiment. Then, after the measured distance is displayed in S22, the tip position detection unit 25 detects the position of the tip of the puncture needle 15 on the photoacoustic image and the movement detection unit 32 checks whether the position of the tip detected by tip position detection unit 25 has been changed. Then, in a case in which the position of the tip of the puncture needle 15 has not been changed on the photoacoustic image (S70, NO), the distance is not remeasured and the measurement result is not updated (S78).

On the other hand, in a case in which the position of the tip of the puncture needle 15 has been changed on the photoacoustic image (S70, YES), the movement detection unit 32 checks whether there is a change in an ultrasound image of a frame immediately after the photoacoustic image (S72). The reason why a change in the ultrasound image is checked in a case in which the position of the tip of the puncture needle 15 has been changed on the photoacoustic image is that the position of the tip of the puncture needle 15 is moved in the photoacoustic image even in a case in which not the puncture needle 15 but the probe 11 is moved with respect to the subject M. In a case in which the probe 11 is moved with respect to the subject, image information is changed not only in the photoacoustic image but also in the ultrasound image. Therefore, a change in the image information in the ultrasound image is checked to identify whether the position of the tip of the puncture needle 15 or the probe 11 has been moved with respect to the subject M.

In a case in which it is checked in S72 that there is a change in the image information in the ultrasound image (S72, YES), it is determined that not the position of the tip of the puncture needle 15 but the probe 11 has been moved with respect to the subject M, the distance is not remeasured, and the measurement result is not updated (S78). On the other hand, in a case in which it is checked in S72 that there is no change in the image information in the ultrasound image (S72, NO), it is determined that not the probe 11 but the position of the tip of the puncture needle 15 has been moved and the distance between the measurement point and the moved position of the tip of the puncture needle 15 is remeasured (S74). Then, the measurement result which is currently displayed is updated with the remeasurement result and the remeasurement result is displayed (S76).

In the above description, the movement detection unit 32 is provided in the photoacoustic image generation apparatus 10 according to the first embodiment. However, the movement detection unit 32 may be provided in the photoacoustic image generation apparatuses 10 according to the second and third embodiments and the same process as described above may be performed.

In the above-described embodiments, the puncture needle 15 is used as an embodiment of the insert. However, the invention is not limited thereto. The insert may be a radio-frequency ablation needle including an electrode that is used for radio-frequency ablation, a catheter that is inserted into a blood vessel, or a guide wire for a catheter that is inserted into a blood vessel. Alternatively, the insert may be an optical fiber for laser treatment.

The insert according to the invention is not limited to a needle, such as an injection needle, and may be a biopsy needle used for biopsy. That is, the needle may be a biopsy needle that is inserted into an inspection target of the living body and extracts the tissues of a biopsy site of the inspection target. In this case, photoacoustic waves may be generated from an extraction portion (intake port) for sucking and extracting the tissues of the biopsy site. In addition, the needle may be used as a guiding needle that is used for insertion into a deep part, such as a part under the skin or an organ inside the abdomen.

The invention has been described above on the basis of the preferred embodiments. However, the insert and the photoacoustic measurement device according to the invention are not limited only to the above-described embodiments.

### Explanation of References

- 10:: photoacoustic image generation apparatus
- 11:: probe
- 12:: ultrasound unit
- 13:: laser unit
- 15:: puncture needle
- 15a:: puncture needle main body
- 15b:: optical fiber
- 15c:: photoacoustic wave generation portion
- 15d:: hollow portion
- 16:: optical cable
- 20:: receiving circuit
- 21:: receiving memory
- 22:: data demultiplexing unit
- 23:: photoacoustic image generation unit
- 24:: ultrasound image generation unit
- 25:: tip position detection unit
- 26:: display control unit
- 27:: transmission control circuit
- 28:: control unit
- 28a:: measurement unit
- 29:: measurement point designation receiving unit
- 30:: image display unit
- 31:: feature point detection unit
- 32:: movement detection unit
- 40:: input unit
- M:: subject
- P1: tip: position of puncture needle
- P2, P3:: measurement point

## Claims

1. A photoacoustic image generation apparatus (10) comprising:
an insert of which at least a tip portion is insertable into a subject and which includes a light guide member that guides light to the tip portion and a photoacoustic wave generation portion (15c) that absorbs the light guided by the light guide member and generates photoacoustic waves;
a probe (11) that detects the photoacoustic waves generated from the photoacoustic wave generation portion (15c) and reflected acoustic waves reflected by transmission of acoustic waves to the subject;
a photoacoustic image generation unit (23) that generates a photoacoustic image on the basis of the photoacoustic waves;
a reflected acoustic image generation unit (24) that generates a reflected acoustic image on the basis of the reflected acoustic waves;
a measurement point designation receiving unit (29) that receives designation of a measurement point (P2, P3) on the reflected acoustic image;
a tip position detection unit (25) that detects a position of a tip of the insert included in the photoacoustic image on the basis of the photoacoustic image; and
a measurement unit (28a) that measures a distance between the measurement point (P2, P3) on the reflected acoustic image and the position (P1) of the tip of the insert detected on the photoacoustic image;
wherein the measurement point designation receiving unit (29) receives the designation of the measurement point (P2, P3) only in a case in which the tip position detection unit (25) detects the position (P1) of the tip of the insert.

2. The photoacoustic image generation apparatus according to claim 1, further comprising:
a feature point detection unit (31) that detects a feature point on the basis of the reflected acoustic image,
wherein the measurement unit (28a) measures a first distance between the position (P1) of the tip of the insert and the designated measurement point (P2, P3) and a second distance between the position (P1) of the tip of the insert and the feature point.

3. The photoacoustic image generation apparatus according to claim 1, further comprising:
a feature point detection unit (31) that detects a feature point on the basis of the reflected acoustic image,
wherein the measurement unit (28a) measures the distance, using the feature point as the measurement point (P2, P3).

4. The photoacoustic image generation apparatus according to any one of claims 1 to 3, further comprising:
a movement detection unit (32) that detects movement of the position (P1) of the tip of the insert, using the reflected acoustic image and the photoacoustic image.

5. The photoacoustic image generation apparatus according to claim 4,
wherein the measurement unit (28a) remeasures the distance in a case in which the movement detection unit (32) detects the movement of the position (P1) of the tip of the insert.

6. The photoacoustic image generation apparatus according to any one of claims 1 to 5,
wherein the probe (11) alternately detects the photoacoustic waves and the reflected acoustic waves.

7. A photoacoustic image generation apparatus, comprising:
an insert of which at least a tip portion is insertable into a subject and which includes a light guide member that guides light to the tip portion and a photoacoustic wave generation portion (15c) that absorbs the light guided by the light guide member and generates photoacoustic waves;
a probe (11) that detects the photoacoustic waves generated from the photoacoustic wave generation portion (15c) and reflected acoustic waves reflected by transmission of acoustic waves to the subject;
a photoacoustic image generation unit (23) that generates a photoacoustic image on the basis of the photoacoustic waves;
a reflected acoustic image generation unit (24) that generates a reflected acoustic image on the basis of the reflected acoustic waves;
a tip position detection unit (25) that detects a position of a tip of the insert included in the photoacoustic image on the basis of the photoacoustic image; and
a measurement unit (28a) that measures a distance between a measurement point (P2, P3) on the reflected acoustic image and the position (P1) of the tip of the insert detected on the photoacoustic image;
a display control unit (26) that displays the distance measured by the measurement unit on a display unit (30),
wherein the display control unit (26) does not display the distance in a case in which the position (P1) of the tip of the insert is not detected after the tip position detection unit (25) detects the position (P1) of the tip of the insert.

## Patentansprüche

1. Photoakustische Bilderzeugungsvorrichtung (10), umfassend:
ein Einführteil, von dem zumindest ein Spitzenabschnitt in ein Subjekt einführbar ist, und das ein Lichtleitelement, welches Licht zu dem Spitzenabschnitt leitet, und einen photoakustischen Wellenerzeugungsabschnitt (15c) enthält, der das von dem Lichtleitelement geleitete Licht absorbiert und photoakustische Wellen erzeugt;
eine Sonde (11), die die von dem photoakustischen Wellenerzeugungsabschnitt (15c) erzeugten photoakustischen Wellen, und reflektierte akustische Wellen, die durch Senden von akustischen Wellen auf das Subjekt reflektiert werden, nachweist;
eine Photoakustikbild-Erzeugungseinheit (23), die ein photoakustisches Bild auf der Grundlage der photoakustischen Wellen erzeugt;
eine Reflexions-Akustikbild-Erzeugungseinheit (24), die auf der Grundlage der reflektierten akustischen Wellen ein Reflexions-Akustikbild erzeugt;
eine Kennzeichnungs-Empfangseinheit (29), die eine Kennzeichnung eines Messpunkts (P2, P3) auf dem Reflexions-Akustikbild empfängt;
eine Spitzenort-Nachweiseinheit (25), die einen Ort einer Spitze des Einführteils in dem Photoakustikbild auf der Grundlage des Photoakustikbilds nachweist; und
eine Messeinheit (28a), die einen Abstand zwischen dem Messpunkt (P2, P3) in dem Reflexions-Akustikbild und dem Ort (P1) der Spitze des Einführteils, der in dem Photoakustikbild nachgewiesen wurde, misst;
wobei die Messpunktkennzeichnungs-Empfangseinheit (29) die Kennzeichnung des Messpunkts (P2, P3) nur in dem Fall empfängt, dass die Spitzenort-Nachweiseinheit (25) den Ort (P1) der Spitze des Einführteils nachweist.

2. Vorrichtung nach Anspruch 1, weiterhin umfassend:
eine Merkmalspunkt-Nachweiseinheit (31), die einen Merkmalspunkt auf der Grundlage des Reflexions-Akustikbilds nachweist,
wobei die Messeinheit (28a) einen ersten Abstand zwischen dem Ort (P1) der Spitze des Einführteils und dem gekennzeichneten Messpunkt (P2, P3) und einen zweiten Abstand zwischen dem Ort (P1) der Spitze des Einführteils und dem Merkmalspunkt misst.

3. Vorrichtung nach Anspruch 1, weiterhin umfassend:
eine Merkmalspunkt-Nachweiseinheit (31), die einen Merkmalspunkt auf der Grundlage des Reflexions-Akustikbilds nachweist,
wobei die Messeinheit (28a) den Abstand unter Verwendung des Merkmalspunkts als den Messpunkt (P2, P3) misst.

4. Vorrichtung nach einem der Ansprüche 1 bis 3, weiterhin umfassend:
eine Bewegungsnachweiseinheit (32), die eine Bewegung des Orts (P1) der Spitze des Einführteils unter Verwendung des Reflexions-Akustikbilds und des photoakustischen Bilds nachweist.

5. Vorrichtung nach Anspruch 4,
bei der die Messeinheit (28a) den Abstand für den Fall erneut misst, dass die Bewegungsnachweiseinheit (32) die Bewegung des Orts (P1) der Spitze des Einführteils nachweist.

6. Vorrichtung nach einem der Ansprüche 1 bis 5,
bei der die Sonde (11) abwechselnd die photoakustischen Wellen und die reflektierten akustischen Wellen nachweist.

7. Photoakustikbild-Erzeugungsvorrichtung, umfassend:
ein Einführteil, von dem zumindest ein Spitzenabschnitt in ein Subjekt einführbar ist, und das ein Lichtleitelement, welches Licht zu dem Spitzenabschnitt leitet, und einen photoakustischen Wellenerzeugungsabschnitt (15c) enthält, der das von dem Lichtleitelement geleitete Licht absorbiert und photoakustische Wellen erzeugt;
eine Sonde (11), die die von dem photoakustischen Wellenerzeugungsabschnitt (15c) erzeugten photoakustischen Wellen, und reflektierte akustische Wellen, die durch Senden von akustischen Wellen auf das Subjekt reflektiert werden, nachweist;
eine Photoakustikbild-Erzeugungseinheit (23), die ein photoakustisches Bild auf der Grundlage der photoakustischen Wellen erzeugt;
eine Reflexions-Akustikbild-Erzeugungseinheit (24), die auf der Grundlage der reflektierten akustischen Wellen ein Reflexions-Akustikbild erzeugt;
eine Spitzenort-Nachweiseinheit (25), die einen Ort einer Spitze des Einführteils in dem photoakustischen Bild auf der Grundlage des photoakustischen Bilds nachweist; und
eine Messeinheit (28a), die einen Abstand zwischen einem Messpunkt (P2, P3) in dem Reflexions-Akustikbild und dem Ort (P1) der Spitze des Einführteils, der in dem Photoakustikbild nachgewiesen wurde, misst;
eine Anzeigesteuereinheit (26), die den von der Messeinheit gemessenen Abstand auf einer Anzeigeeinheit (30) anzeigt,
wobei die Anzeigesteuereinheit (26) den Abstand dann nicht anzeigt, wenn der Ort (P1) der Spitze des Einführteils nicht nachgewiesen wird, nachdem die Spitzenort-Nachweiseinheit (25) den Ort (P1) der Spitze des Einführteils nachgewiesen hat.

## Revendications

1. Appareil de génération d'image photoacoustique (10), comprenant :
un insert dont au moins une portion de pointe peut être introduite dans un sujet et lequel inclut un élément de guide optique, lequel guide la lumière vers la portion de pointe, et une portion de génération d'ondes photoacoustiques (15c), laquelle absorbe la lumière guidée par l'élément de guide optique et génère des ondes photoacoustiques ;
une sonde (11), laquelle détecte les ondes photoacoustiques générées à partir de la portion de génération d'ondes photoacoustiques (15c) et des ondes acoustiques réfléchies, réfléchies par transmission d'ondes acoustiques vers le sujet ;
une unité de génération d'image photoacoustique (23), laquelle génère une image photoacoustique sur la base des ondes photoacoustiques ;
une unité de génération d'image acoustique réfléchie (24), laquelle génère une image acoustique réfléchie sur la base des ondes acoustiques réfléchies ;
une unité de réception de désignation de point de mesure (29), laquelle reçoit une désignation de point de mesure (P2, P3) sur l'image acoustique réfléchie ;
une unité de détection de position de pointe (25), laquelle détecte une position d'une pointe de l'insert incluse dans l'image photoacoustique sur la base de l'image photoacoustique, et
une unité de mesure (28a), laquelle mesure une distance entre le point de mesure (P2, P3) sur l'image acoustique réfléchie et la position (P1) de la pointe de l'insert détectée sur l'image photoacoustique,
dans lequel l'unité de réception de désignation de point de mesure (29) reçoit la désignation du point de mesure (P2, P3) uniquement dans un cas où l'unité de détection de position de pointe (25) détecte la position (P1) de la pointe de l'insert.

2. Appareil de génération d'image photoacoustique selon la revendication 1, comprenant en outre :
une unité de détection de point de caractéristique (31), laquelle détecte un point de caractéristique sur la base de l'image acoustique réfléchie,
dans lequel l'unité de mesure (28a) mesure une première distance entre la position (P1) de la pointe de l'insert et le point de mesure désigné (P2, P3), et une seconde distance entre la position (P1) de la pointe de l'insert et le point de caractéristique.

3. Appareil de génération d'image photoacoustique selon la revendication 1, comprenant en outre :
une unité de détection de point de caractéristique (31), laquelle détecte un point de caractéristique sur la base de l'image acoustique réfléchie,
dans lequel l'unité de mesure (28a) mesure la distance, à l'aide du point de caractéristique comme point de mesure (P2, P3).

4. Appareil de génération d'image photoacoustique selon l'une quelconque des revendications 1 à 3, comprenant en outre :
une unité de détection de déplacement (32), laquelle détecte le déplacement de la position (P1) de la pointe de l'insert, à l'aide de l'image acoustique réfléchie et de l'image photoacoustique.

5. Appareil de génération d'image photoacoustique selon la revendication 4,
dans lequel l'unité de mesure (28a) mesure à nouveau la distance dans un cas où l'unité de détection de déplacement (32) détecte le déplacement de la position (P1) de la pointe de l'insert.

6. Appareil de génération d'image photoacoustique selon l'une quelconque des revendications 1 à 5,
dans lequel la sonde (11) détecte par alternance les ondes photoacoustiques et les ondes acoustiques réfléchies.

7. Appareil de génération d'image photoacoustique, comprenant :
un insert dont au moins une portion de pointe peut être introduite dans un sujet et lequel inclut un élément de guide optique, lequel guide la lumière vers la portion de pointe, et une portion de génération d'ondes photoacoustiques (15c), laquelle absorbe la lumière guidée par l'élément de guide optique et génère des ondes photoacoustiques ;
une sonde (11), laquelle détecte les ondes photoacoustiques générées à partir de la portion de génération d'ondes photoacoustiques (15c) et des ondes acoustiques réfléchies, réfléchies par transmission d'ondes acoustiques vers le sujet ;
une unité de génération d'image photoacoustique (23), laquelle génère une image photoacoustique sur la base des ondes photoacoustiques ;
une unité de génération d'image acoustique réfléchie (24), laquelle génère une image acoustique réfléchie sur la base des ondes acoustiques réfléchies ;
une unité de détection de position de pointe (25), laquelle détecte une position d'une pointe de l'insert incluse dans l'image photoacoustique sur la base de l'image photoacoustique, et
une unité de mesure (28a), laquelle mesure une distance entre le point de mesure (P2, P3) sur l'image acoustique réfléchie et la position (P1) de la pointe de l'insert détectée sur l'image photoacoustique,
une unité de commande d'affichage (26), laquelle affiche la distance mesurée par l'unité de mesure sur l'unité d'affichage (30),
dans lequel l'unité de commande d'affichage (26) n'affiche pas la distance dans un cas où la position (P1) de la pointe de l'insert n'est pas détectée après que l'unité de détection de position de pointe (25) détecte la position (P1) de la pointe de l'insert.
